(19) Europäisches Patentamt
European Patent Office
Office européen des brevets

(11) Publication number: **0 287 523 B1**

# (12) EUROPEAN PATENT SPECIFICATION

(45) Date of publication of patent specification: **09.06.93**

(51) Int. Cl.$^5$: **C07B 57/00**, C07C 237/00

(21) Application number: **88830107.4**

(22) Date of filing: **18.03.88**

The file contains technical information submitted after the application was filed and not included in this specification

## (54) Process for producing (-) N-acetylaminocarnitine and (+) N-acetylaminocarnitine.

(30) Priority: **18.03.87 IT 4773887**

(43) Date of publication of application:
**19.10.88 Bulletin 88/42**

(45) Publication of the grant of the patent:
**09.06.93 Bulletin 93/23**

(84) Designated Contracting States:
**AT BE CH DE ES FR GB GR LI LU NL SE**

(56) References cited:
**EP-A- 0 080 695**
**EP-A- 0 127 098**
**WO-A-85/04396**

(73) Proprietor: **SIGMA TAU Industrie Farmaceutiche Riunite S.p.A.**
**Viale Shakespeare, 47**
**I-00144 Rome(IT)**

(72) Inventor: **Tinti, Maria Ornella**
**Via Ernesto Basile, 81**
**I-00128 Rome(IT)**
Inventor: **Misiti, Domenico**
**Via Bacchiglione, 3**
**I-00199 Rome(IT)**

(74) Representative: **Fassi, Aldo, Dr.**
**c/o Sigma-Tau Industrie Farmaceutiche**
**Riunite S.p.A. Via Pontina, Km. 30, 400**
**I-00040 Pomezia (Roma) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to a process for producing (-) N-acetylaminocarnitine and (+) N-acetylaminocarnitine by resolution of a racemic mixture of (±) N-acetylaminocarnitine.

Recently, the discovery of certain pharmacological properties of aminocarnitine ((I), wherein R = H) and some alkanoylaminocarnitines ((I), wherein R = acetyl, decanoyl, palmitoyl)

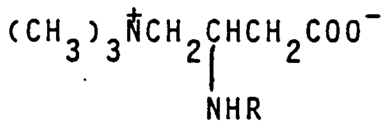

(I)

has promoted considerable interest in these compounds. For instance, D.L. Jenkins and O.W. Griffith have disclosed the antiketogenic and hypoglycemic effects of the racemates of the compounds (I). Also in the US patent 4,521,432 to Takeda there is disclosed the antiketogenic activity (with attendant perspective of utilization in the therapeutical treatment of diabetes) of the optically active (-) N-acetylaminocarnitine inner salt ($[\alpha]_D^{25}$ = - 17,4, C = 1, $H_2O$) obtained by cultivation of a microorganism of the genus Emericella or Aspergillus, or via a complex chemical process. Also (+) aminocarnitine chloride hydrochloride ($[\alpha]_D^{25}$ = + 6,3, C = 1, AcOH 1N) obtained by hydrolyzing the above-mentioned optically active acetylderivative, is reported to possess similar activity.

There has been, therefore, a demand for a process suitable to provide both enantiomeric forms of N-acetylaminocarnitine, whose absolute configuration is, incidentally, still unknown.

According to the present invention, the process for producing (-) N-acetylaminocarnitine inner salt and (+) N-acetylaminocarnitine inner salt by resolution of a racemic mixture of (±) N-acetylaminocarnitine inner salt is essentially characterized by the steps of:

(1) reacting (±) N-acetylaminocarnitine inner salt with an optically active acid selected from (+) dibenzoyl D(-) tartaric acid and (-) dibenzoyl L(+) tartaric acid, thus obtaining optically active salts consisting of the diastereoisomers (+) dibenzoyl D(-) tartrate of (+) N-acetyl aminocarnitine and (+) dibenzoyl D(-) tartrate of (-) N-acetylaminocarnitine, or, respectively, the diastereoisomers (-) dibenzoyl L(+) tartrate of (+) N-acetylcarnitine and (-) dibenzoyl L(+) tartrate of (-) N-acetylaminocarnitine; and

(2) separating the diastereoisomers by fractional crystallization.

The starting compound (±) N-acetylaminocarnitine inner salt can be synthesized e.g. via the method disclosed by D. Jenkins in J. Biol.Chem. 260/27, 14.748, 1985. When (+) dibenzoyl D(-) tartaric acid is used as resolution agent,(+) dibenzoyl D(-) tartrate of (-) N-acetylaminocarnitine precipitates whereas its diastereoisomer remains dissolved in the mother liquor wherefrom it can be easily isolated via known procedures.

When (-) dibenzoyl L(+) tartaric acid is used as resolution agent, (-) dibenzoyl L(+) tartrate of (+) N-acetylaminocarnitine precipitates, whereas its diastereoisomer remains dissolved in the mother liquor.

(-) N-acetylaminocarnitine and (+) N-acetylaminocarnitine are isolated from the respective diastereoisomers via known procedures.

(-) aminocarnitine and (+) aminocarnitine can be easily obtained via acid hydrolysis of the corresponding optical antipodes, (+) and (-) N-acetylaminocarnitine, respectively. The following non limiting examples illustrate the process of the present invention.

### EXAMPLE 1

(-)dibenzoyl L(+) tartaric acid (12 g; 0.032 moles) and (±) N-acetylaminocarnitine inner salt (6,5 g; 0.032 moles) were dissolved in an amount of methanol sufficient to solubilize both reagents.

The resulting solution was concentrated under vacuum to dryness.

A solid product (18.5 g) which had optical rotatory power $[\alpha]_D^{25}$ = - 80.5 (C = 1,$H_2O$)) was obtained. This solid was taken up with 80 ml of methanol at room temperature and kept at 6 °C for 15 days. A solid product crystallized which showed optical rotatory power $[\alpha]_D^{25}$ = -75.8.

Its HPLC analysis showed that it consisted of N-acetylaminocarnitine and dibenzoyl tartaric acid (ratio 1:1).

Chromatograph Waters

Column $\mu$ Bondapak -$NH_2$

eluent $KH_2PO_4$ 0,05M-$CH_3CN$(35-65)

detector UV HP 1040A = $\lambda$ 205 attenuation 30

flow rate 1 ml/min
chart speed 0,5 cm/min
dibenzoyl tartaric acid $R_t$ = 4,03
N-acetylaminocarnitine = 10,5

This solid (3.9 g) was dissolved in 20 ml of $H_2O$ and the resulting aqueous solution was extracted with 50 ml of ethyl ether in order to remove the benzoyl tartaric acid. (It was thus possible to recover the resolution agent from the organic phase).

The aqueous solution was eluted on a strongly anionic ion exchange resin IRA 402 activated in the form $HCO_3^-$ (or $OH^-$) in order to remove possible traces of unreacted dibenzoyl tartaric acid. The collected eluate was lyophilized. On gram of (+) N-acetylaminocarnitine inner salt was obtained which had opical rotatory power $[\alpha]_D^{25}$ = + 21 (C = $H_2O$) melting point = 187-188 °C; NMR corresponding.
This product was hydrolized with 25 ml of 5N HCl for 24 hours at 90 °C.

The reaction mixture was then brought to dryness and the residue was crystallized from methanol. 500 mg of (-) amino carnitine chloride hydrochloride were obtained.
M.P. 235-236 °C
$[\alpha]_D^{25}$ = - 7,45 (C = 1 $H_2O$)

NMR $D_2O$ δ 4,3 (1H, m, -CH-); 3,9 (2H, d, $N-CH_2-$);
3,3 (9H, s, $(CH_3)_3N-$); 3,1 (2H, d, $-CH_2COOH$).

HPLC
Chromatograph Waters
Column μ Bondapak - $NH_2$
eluent $KH_4PO_4$ 0,05M-$CH_3CN$ (35-65)
detector UV HP 1040A = 205 attenuation 30
flowrate 1 ml/min
chart speed 0.5 cm/min
$R_t$ = 20.365

EXAMPLE 2

(+) dibenzoyl D(-) tartaric acid (19.5 g; 0.052 moles) and (±) N-acetylaminocarnitine inner salt (10.6 g; 0.052 moles) were dissolved in an amount of methanol sufficient to solubilized the reagents. The resulting solution was concentrated under vacuum to dryness. A solid product (30.1 g) was obtained which had optical rotatory power $[\alpha]_D^{25}$ = + 80 (C = 1, $H_2O$). This solid was taken up with 150 ml of methanol at 50 °C. Subsequently, the solution was kept for 6 days at room temperature. A solid product crystallized with optical rotatory power $[\alpha]_D^{25}$ = + 77 (C 1, $H_2O$), which via HPLC analysis was shown to consist of N-acetylaminocarnitine and dibenzoyl tartaric acid at ratio 1:1. (HPLC as described in example 1).

This solid (7.3 g) was dissolved in 40 ml of $H_2O$ and extracted with 50 ml of ethyl ether in order to remove unreacted dibenzoyl tartaric acid.

The acqueous solution was eluted on IRA 402 resin activated in a $HCO_3^-$ (or $OH^-$) form in order to remove the unreacted tartaric acid and then lyophilized. The lyophilized product (2.3 g) consisted of (-) N-acetylaminocarnitine inner salt.
$[\alpha]_D^{25}$ = - 20,4 (C = 1 $H_2O$)
Melting Point = 188-190 °C
NMR responding

(-) N-acetylaminocarnitine was hydrolyzed with HCl as described in example 1. (+) aminocarnitine was obtained .
$[\alpha]_D^{25}$ = + 7,07 (C = 1, $H_2O$)
Melting point was 227-229 °C
NMR responding HPLC as described in example 1.

## Claims

1. A process for producing (-) N-acetylaminocarnitine inner salt and (+) N-acetylaminocarnitine inner salt by resolution of a racemic mixture of (±) N-acetylaminocarnitine inner salt, characterized by the steps of:

    (1) reacting (±) N-acetylaminocarnitine inner salt with an optically active acid selected from (+) dibenzoyl D(-) tartaric acid and (-) dibenzoyl L(+) tartaric acid, thus obtaining optically active salts consisting of the diastereoisomers (+) dibenzoyl D(-) tartrate of (+) N-acetylaminocarnitine and (+) dibenzoyl D(-) tartrate of (-) N-acetylaminocarnitine, or, respectively, the diastereoisomers (-) dibenzoyl L(+) tartrate of (+) N-acetylaminocarnitine and (-) dibenzoyl L(+) tartrate of (-)N-acetylaminocarnitine
    (2) separating the diastereoisomers by fractional crystallization; and
    (3) isolating (-) N-acetylaminocarnitine and (+) N-acetylaminocarnitine from the respective diastereoisomers via known procedures.

## Patentansprüche

1. Verfahren zur Herstellung von
    (-) N-Acetylaminocarnitin-inneres Salz und
    (+) N-Acetylaminocarnitin-inneres Salz durch Aufspaltung eines racemischen Gemisches von
    (±) N-Acetylaminocarnitin-inneres Salz,
    **gekennzeichnet** durch die Schritte:
    (1) Umsetzung von (±) N-Acetylaminocarnitin-inneres Salz mit einer optisch aktiven Säure, ausgewählt aus
    (+) Dibenzoyl D(-) Weinsäure und
    (-) Dibenzoyl L(+) Weinsäure, wodurch optisch aktive Salze, bestehend aus den Diastereomeren
    (+) Dibenzoyl D(-) Tartrat von (+) N-Acetylaminocarnitin und (+) Dibenzoyl D(-) Tartrat von
    (-) N-Acetylaminocarnitin bzw. den Diastereomeren
    (-) Dibenzoyl L(+) Tartrat von (+) N-Acetylaminocarnitin und (-) Dibenzoyl L(+) Tartrat von
    (-) N-Acetylaminocarnitin, erhalten werden;
    (2) Trennung der Diastereomere durch fraktionierte Kristallisation; und
    (3) Isolierung von (-) N-Acetylaminocarnitin und
    (+) NAcetylaminocarnitin aus den jeweiligen Diastereomeren durch bekannte Verfahren.

## Revendications

1. Procédé de production de sel interne de la (-)N-acétylaminocarnitine et de sel interne de la (+) N-acétylaminocarnitine par résolution d'un mélange racémique du sel interne de (±) N-acétylaminocarnitine caractérisé par les étapes Suivantes :

    (1) réaction du sel interne de (±)N-acétylaminocarnitine avec un acide optiquement actif choisi parmi l'acide (+)dibenzoyl D(-) tartrique ou l'acide (-)dibenzoyl L(+)tartrique permettant d'obtenir des sels optiquement actifs qui consistent en diastéréoisomères du D(-)tartrate de (+)dibenzoyl de (+)N-acétylaminocarntine et le D(-)tartrate de (+)dibenzoyl de (-)N'acétylaminocarnitine ou, respectivement, en diastéréoisomères de L(+)tartrate de (-)dibenzoyl de (+)N-aminoacétylcarnitine et en L-(+)tartrate de (-)dibenzoyl de (-)N-acétylaminocarnitine
    (2) séparation des diastéréoisomères par cristallisation fractionnée ; et
    (3) Purification de la (-)N-acétylaminocarnitine et de la (+)N-acétylaminocarnitine à partir des diastéréoisomères respectifs au moyen de procédés connus.